# EUROPEAN PATENT APPLICATION

(11) **EP 4 215 615 A1**
(43) Date of publication of application: **26.07.2023**
(21) Application number: 22152265.9
(22) Date of filing: 19.01.2022
(51) Int. Cl.: C12P 7/22, C12P 1/02, A23K 10/12, A23K 50/90

(54) **PROCESS FOR THE PRODUCTION OF AN INSECT SUBSTRATE, INSECT SUBSTRATE AND USES THEREOF**

(71) Applicant: Fraunhofer-Gesellschaft zur Förderung der angewandten Forschung e.V., 80686 München (DE)
(72) Inventor: Klüber, Patrick, 35392 Gießen (DE); Zorn, Holger, 35392 Gießen (DE); Rühl, Martin, 35392 Gießen (DE); Pfeiffer, Janin, 35392 Gießen (DE); Bakonyi, Daniel, 35392 Gießen (DE); Vilcinskas, Andreas, 35392 Gießen (DE)
(74) Representative: Pfenning, Meinig & Partner mbB

(57) **Abstract**

A process for the production of an insect substrate is provided, which comprises or consists of the steps of sterilizing a substrate comprising lignocellulose, mixing the sterilized substrate comprising lignocellulose with a lignin-degrading fungus to form a solid state mixture and incubating the solid state mixture to allow a solid state fermentation, wherein enzymes produced by the fungus convert the solid state mixture to an insect substrate. The process is characterized in that it does not comprise a step in which the substrate comprising lignocellulose is subjected to a delignification treatment involving an addition of NaOH to the substrate comprising lignocellulose before the solid state fermentation. The process has the advantage that it allows an easier, faster, more efficient, lower-cost and more sustainable and industrial-scale production of an insect substrate from a substrate comprising lignocellulose than known processes. Moreover, an insect substrate is provided and uses thereof are proposed.

## Description

A process for the production of an insect substrate is provided, which comprises or consists of the steps of sterilizing a substrate comprising lignocellulose, mixing the sterilized substrate comprising lignocellulose with a lignin-degrading fungus to form a solid state mixture and incubating the solid state mixture to allow a solid state fermentation, wherein enzymes produced by the fungus convert the solid state mixture to an insect substrate. The process is characterized in that it does not comprise a step in which the substrate comprising lignocellulose is subjected to a delignification treatment involving an addition of NaOH to the substrate comprising lignocellulose before the solid state fermentation. The process has the advantage that it allows an easier, faster, more efficient, lower-cost and more sustainable and industrial-scale production of an insect substrate from a substrate comprising lignocellulose than known processes. Moreover, an insect substrate is provided and uses thereof are proposed.

Insects offer a promising alternative source of protein to mitigate the environmental consequences of conventional livestock farming. For example, it is known that larvae of the black soldier fly (*Hermetia illucens,* BSF) can efficiently convert a variety of organic side streams into valuable proteins, lipids, and chitin (Smetana, S. et al., Resources, Conservation and Recycling, vol. 144, p. 285-296.). In 2017, the EU allowed the use of insects as aquaculture feed and, in 2021, as feed for pigs and poultry, likely increasing the future demand for insect biomass. Globally meaningful insect production, however, requires substrates that are available at large volume while satisfying nutritional and economic considerations.

The growing worldwide demand for proteins and lipids contrasts with the inefficient use of agro industrial raw materials and the modern throwaway society. Large amounts of underutilized organic material accumulate as side streams in many parts of the agri-food industry, including palm oil production to meet the increasing demand for vegetable oils. In 2018, 71.5 million tons of crude palm oil was produced on an area of 18.9 million ha (Andlar, M. et al., Engineering in Life Sciences, vol., 18, p. 768-778). During the production of crude palm oil, a milling process and subsequent refining and fractionation steps are employed which produce side streams such as empty fruit bunches (EFB), palm kernel meal (PKM), as well as sludge and mill effluents (POME), amounting to many millions of tons of organic material per year. Importantly, the disposal of palm oil side products is a major environmental problem, often said side products are burnt as a means of disposal although other more purposeful solutions as biofuel or fertilizer are possible (see e.g. WO 2015/108409 A1), but not yet realizable on industrial scale (see e.g. Dickinson, E. et al., PLOS ONE, vol. 14, e0224771).

However, palm oil production side streams, particularly empty fruit bunches (EFB), have not been used for an effective and industrial-scale insect production, although the high sugar content of lignocellulose containing palm oil side streams, like EFB, makes them an interesting substrate for insect production (see e.g. US 2017/311612 A1). However, the utilization of EFB is challenging due to its high content of lignocellulose. Unlocking all palm oil side products including EFB as substrates for a large-scale insect biomass production would enable an upcoming animal biomass production sector with a financially highly rewarding feed.

While chemical and mechanical pre-processing methods (including steaming, pressure cooking, composting, microwaving, and breaking down the EFB using ionic liquids) have been tested for providing insect feed from palm oil side products, these methods are inefficient, costly, and time consuming (Dickinson, E. et al., PLOS ONE, vol. 14, e0224771).

It is known in the prior art that the compact structure of lignocelluloses can be broken down by enzyme systems which are present in some microorganisms performing anaerobic digestion, e.g. in certain wood-degrading fungi (Andlar, M. et al., Engineering in Life Sciences, vol., 18, p. 768-778).

The white-rot fungus *Phanerocheate chrysosporium* has been used in a process for producing ligninase enzyme from a lignin-containing biomass (see MY 142205 A1). However, said process involves a delignification treatment of the substrate before beginning with the solid state fermentation. Said delignification treatment is according to Ghosh and Deb and encompasses soaking the substrate in 1 % (w/v) NaOH for several hours, steaming at 120 °C for 1 hour, washing the material free of alkali and drying the material (see Ghosh, V.K. & Deb, J.K., Applied Microbiology and Biotechnology, vol. 29, p. 44-47). The use of NaOH, steam, water for washing and heat energy for drying is expensive and the incubation periods are time-consuming. Hence, the process is not suitable for an easy, fast, efficient, low-cost (economical) and sustainable production of industrial-scale (i.e. tonne-scale) amounts of an insect substrate.

In view of the above, it was the object of the present invention to provide a process for a production of an insect substrate from a substrate comprising lignocellulose which overcomes the disadvantages of the prior art. Particularly, the process should allow an easier, faster, more efficient, lower-cost and more sustainable industrial-scale production of an insect substrate from a substrate comprising lignocellulose. A further object was the provision of an insect substrate by said process and the proposal of uses thereof.

The object is solved by the process having the features of claim 1, the insect substrate having the features of claim 14 and the use of the insect substrate having the features of claim 15. The dependent claims show advantageous embodiments.

According to the invention, a process for the production of an insect substrate is provided, the process comprising or consisting of the following steps:
a) Sterilizing a substrate comprising lignocellulose;
b) Mixing the sterilized substrate comprising lignocellulose with a lignin-degrading fungus to form a solid state mixture; and
c) Incubating the solid state mixture to allow a solid state fermentation, wherein enzymes produced by the fungus convert the solid state mixture to an insect substrate;
characterized in that the process does not comprise a step in which the substrate comprising lignocellulose is subjected to a delignification treatment involving an addition of NaOH (sodium hydroxide) to the substrate comprising lignocellulose.

The inventive process allows an easier, faster, lower-cost, more efficient and sustainable industrial-scale production of an insect substrate from a substrate comprising lignocellulose. One reason is that the process according to the invention does not comprise the delignification treatment before performing the solid state fermentation. This renders the process easier and faster because this treatment is omitted. Furthermore, since NaOH is saved, the process becomes less expensive. It has also been discovered that the delignification treatment before carrying out the solid state fermentation is not needed for successful production of a high-quality substrate for growing insects (insect substrate) and that said step would only remove ingredients (lignocellulose and lignin) which a lignin-degrading fungus can use for providing suitable insect feed. Hence, by omitting said step, the process provides a higher-quality insect substrate and thus becomes more efficient in providing an insect substrate than known prior art processes.

In a preferred embodiment of the process, the process does not comprise a step of extracting a ligninase enzyme. In a further preferred embodiment, the process does not comprise adding an ionic liquid. Moreover, it is preferred that the process does not comprise a step of adding an organic solvent. The absence of at least one of these steps, especially all of these steps, allows an easier, faster, safer, more efficient, and less expensive production of insect substrate in industrial scales.

The substrate used in the process can comprises or consists of at least one (preferably unwashed) side stream of palm oil production. Using a side stream of palm oil production as lignin-comprising substrate has the advantage that the side stream of palm oil production is not wasted, but instead converted to a useful substrate suitable for purposefully growing insects in large scale as food source. This is an economic advantage. Furthermore, there is an ecologic advantage because the side stream of palm oil production is not burned like palm oil side streams in processes of the prior art, i.e. a CO₂-release in the atmosphere is reduced.

The substrate preferably comprises or consists of empty fruit bunches (EBF) originating from a side stream of palm oil production. Said EFB have the advantage that they contain a relatively high amount of lignin, which is a useful energy source for a lignin-degrading fungus, and have a relatively high moisture content of approximately 60 % (v/w), which provides ideal conditions for a solid state fermentation. It is preferred that the substrate comprises EBF in an amount of 50 to 90 wt.-%, preferably 60 to 80 wt.-%, more preferably 65 to 75 wt.-%, relative to the total weight of the substrate. Amounts in this range naturally occur in a mixture of different side streams of palm oil production, especially a mixture of an EFB side stream and a palm kernel meal side stream (PKM side stream). Hence, the advantage is that, for obtaining said amount of EFB, EFB and PKM side streams obtained during the production process of palm oil just have to be mixed in the amount they are produced in said process, i.e. without any part of one of these side streams being wasted.

The substrate preferably comprises or consists of palm kernel meal (PKM) originating from a side stream of palm oil production. Said PKM have the advantage that they contain a relatively high amount of fibers and proteins, which represent a useful energy source for a lignin-degrading fungus. It is preferred that the substrate comprises PKM in an amount of 10 to 50 wt.-%, preferably 20 to 40 wt.-%, more preferably 25 to 35 wt.-%, relative to the total weight of the substrate. Amounts in this range naturally occur in a mixture of different side streams of palm oil production, especially a mixture of PKM side stream with an EFB side stream. Thus, the advantage is that, for obtaining said amount of PKM, PKM and EFB side streams obtained during the production process of palm oil just have to be mixed in the amount they are produced in said process, i.e. without any part of one of these side streams being wasted.

In a preferred embodiment, the substrate does not comprise wheat flour (like e.g. in the process of MY 142205 A). Importantly, wheat flour is an important food for human beings and its use as growth substrate for fungi or insects is not economical because it is an expensive ingredient. The omission of its use as substrate for fungi during solid state fermentation provokes that the production of the insect substrate becomes less expensive and more sustainable. Particularly preferably, in the process, no carbon source being different to a side stream of palm oil production is used. The advantage is that a very inexpensive carbon source is used as the substrate, namely a carbon source which is considered to be waste in known processes for palm oil production. The exclusion of other carbon sources in the process renders the process very economical.

In the process, the substrate comprising lignocellulose can be sterilized by steam, preferably by pressured steam at a temperature in the range of >100 °C to 121 °C.

In the process, it is possible that the substrate is present in the form of particles. Alternatively, the substrate can be processed to particles in the process, preferably by a method selected from the group consisting of crushing, pearling, cracking, chopping, shredding, grinding, ball-milling and combinations thereof.

The size of the particles of the substrate is preferably in the range of 0.1 mm to 1 cm, more preferably 0.2 mm to 5 mm, even more preferably 0.5 mm to 2 mm, wherein the particle size refers to a particle size determined by microscopy. A particle size in this range has the advantage that, on the one hand, a large surface area of substrate is accessible for the fungus, which facilitates and accelerates fermentation, and, on the other hand, unnecessary energy for providing smaller substrate particles is saved and clogging of the particles due to a too small particle size is prevented.

The lignin-degrading fungus used in the process can be selected from the group consisting of soft-rot fungi, brown-rot fungi and white-rot fungi. Preferably, the fungus is selected from the group consisting of white-rot fungi.

In a preferred embodiment, the lignin-degrading fungus is a fungus of the division Basidiomycota, preferably a fungus of the class Agaricomycetes. Fungi of this division and class have been found to be particularly efficient in converting side streams of palm oil production (as lignin-containing substrates) to a substrate for growing insects.

The fungus used in the process can be a fungus of the order Agaricales, preferably a fungus of the family Marasmiaceae, particularly a fungus of the genus *Marasmius,* especially a fungus of the species *Marasmius palmivorus.*

Moreover, the fungus used in the process can be a fungus of the order Polyporales, preferably a fungus of the family Phanerochaetaceae or Cerrenaceae, particularly a fungus of the genus *Bjerkandera* or *Cerrena* (synonym: *Irpex*), especially a fungus of the species *Bjerkandera adusta* or *Cerrena consors.* Particularly preferred is a fungus of the species *Bjerkandera adusta* because this fungus species was discovered to convert side streams of palm oil production to a high-quality substrate for growing insects.

In a preferred embodiment of the process, the solid state mixture is adjusted to have a moisture content in the range of 40 to 80 % (v/w), preferably 50 to 70 % (v/w), more preferably 55 to 65 % (v/w), especially 60 % (v/w). A moisture content in said ranges allows a quick start of the solid state fermentation.

By the same token, the mixture is preferably incubated at a relative humidity in the range of 40 to 80 % (v/w), preferably 50 to 70 % (v/w), more preferably 55 to 65 % (v/w), especially 60 % (v/w). This provides perfect conditions for the solid state fermentation and allows a very fast solid state fermentation. If needed, the relative humidity can be adjusted by adding water, more preferably an water vapour in air, to the mixture.

In a further preferred embodiment, the mixture is incubated at a temperature of 20 to 31°C, preferably 22 to 30 °C, more preferably 24 to 29°C, even more preferably 26 to 28°C, especially 28°C. The advantage is that a temperature in this range can be provided by the outside environment in many locations on earth, especially at locations at which palm oil production is conducted and palm oil side streams are provided as lignin-comprising substrates for the process. In other words, since the energy for providing a temperature in said range does not have to be provided by an artificial heat input (e.g. by inputting heat produced by burning fossil fuels or by converting electrical energy to heating energy), the process can be conducted more economically and ecologically than other known processes.

In the process, the mixture can be incubated for at least 16 days, preferably at least 20 days, more preferably at least 24 days, even more preferably 26 to 30 days, especially 28 days. Longer incubation periods ensure that the substrate comprising lignin is converted to a substrate which comprises a high content of ingredients which are suitable for growing insects and a low content of lignin, especially no lignocellulose.

In a preferred embodiment of the invention, incubating the solid state mixture (during solid state fermentation) is not implemented in a flask, especially in no vessel. The advantage is that the substrate comprising lignocellulose (educt) does not have to be put into a flask or vessel and, after the solid state fermentation, the insect substrate (product) does not have to be removed from the flask. Besides saving expenses for large-volume flasks/vessels, this procedure saves time and energy and renders the process more economical.

By the same token, it is preferred that, in the inventive process, incubating the solid state mixture (during solid state fermentation) is not implemented in a rotary drum bioreactor, preferably not implemented in a tray bioreactor, packed bed bioreactor, rotary drum bioreactor, swing solid state bioreactor, stirred vessel bioreactor and air solid fluidized bed bioreactor, more preferably not implemented in any bioreactor. This approach saves expenses for a bioreactor and saves energy which is usually needed for operating bioreactors (e.g. large fermenters). It also saves time because the substrate comprising lignocellulose (educt) does not have to be put into the bioreactor and, after the solid state fermentation, the insect substrate (product) does not have to be removed from the bioreactor.

For avoiding the use of a flask, vessel and/or bioreactor in the process, the substrate comprising lignocellulose can e.g. simply be piled up in the open air (optionally also covered with a planket) to let the solid state fermentation happen. Open air in regions having a favourable climate (e.g. temperature in the range of 20 to 31 °C and relative humidity in the range of 40 to 80 % (v/w)) is particularly suitable.

Furthermore, an insect substrate is provided which is producible with the process according to the invention. Since the process according to the invention does not comprise a step in which the substrate comprising lignocellulose is subjected to a delignification treatment involving an addition of NaOH to the substrate, the solid state fermentation in the inventive process starts with a substrate having a higher lignin content and comprising naturally-occuring lignin and no lignin which has been chemically modified by an incubation with NaOH. It follows that the insect substrate (product) of the inventive process comprises enzymatic degradation products (especially degradation products of lignocellulose or lignin) which are different to the enzymatic degradation products of processes in which the educt is subjected to a delignification treatment involving an addition of NaOH prior to solid state fermentation. The cheap-to-produce insect substrate provided with the inventive method could at least in part cover the demands of the worldwide growing insect production sector for proteins, lipids, and chitin.

Moreover, a use of the insect substrate according to the invention for growing insect larvae, preferably for growing larvae of the black soldier fly, is proposed.

In the following figures and examples, the invention will be explained in more detail without wishing to limit the invention to the specific embodiments shown here.

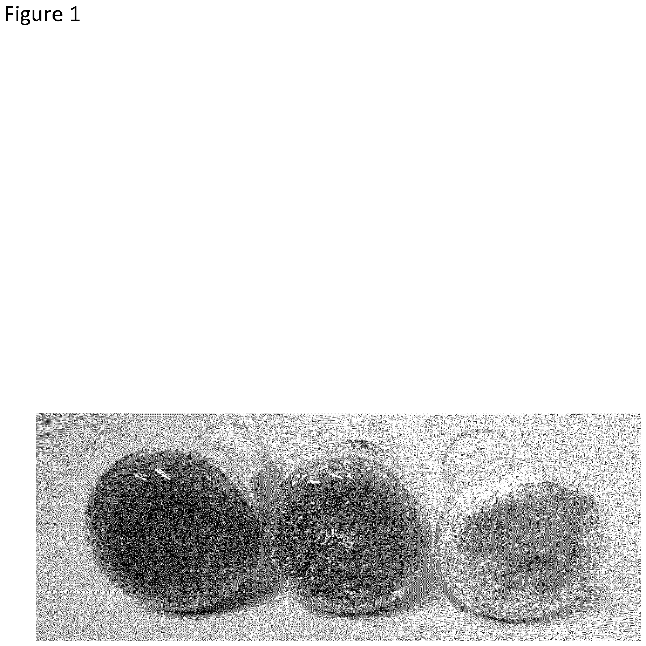

Figure 1 shows the results of a screening for the ability of the white-rot fungus *Bjerkandera adusta* to metabolize a substrate consisting of empty fruit bunches (EFB) originating from a palm oil side stream. Depicted is the fermentative degradation process after 1 week (left picture), 2 weeks (middle picture) and 4 weeks (right picture). A successive bleaching and increase of fungal biomass from the left to the right picture reveals the ability of *B. adusta* to metabolize EFB.

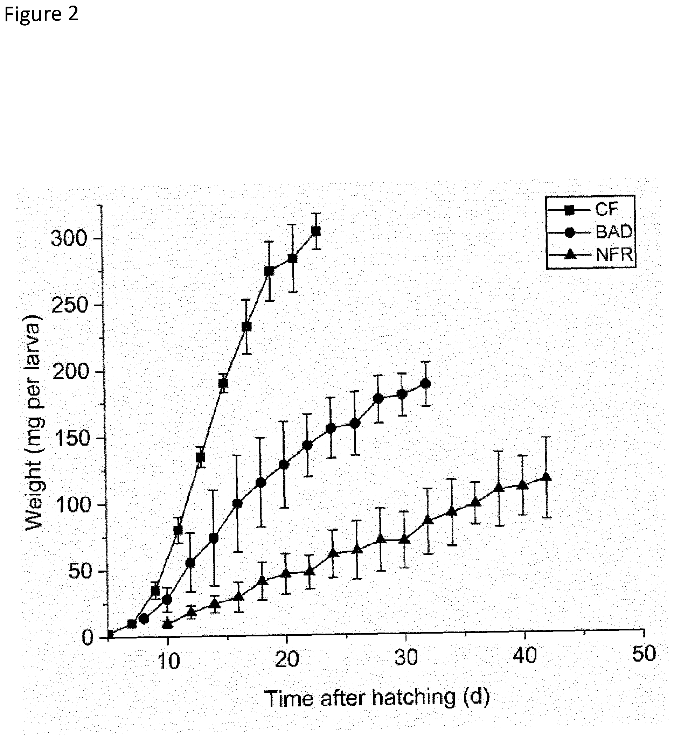

Figure 2 shows the results of an experiment in which BSF larvae were reared on different substrates. Illustrated is the weight of the larvae (which is a measure for their growth) over time (days). The used substrates were chicken feed (abbreviated: "CF"), a mixture of 70 wt.-% EFB and 30 wt.-% PKM fermented by *B. adusta* in solid state fermentation for 28 days (abbreviated: "BAD"), and a non-fermented reference mixture of 70 wt.-% EFB and 30 wt.-% PKM (abbreviated: "NFR"). Data are mean larval weights (± SD) of three replicate boxes per diet (n = 25).

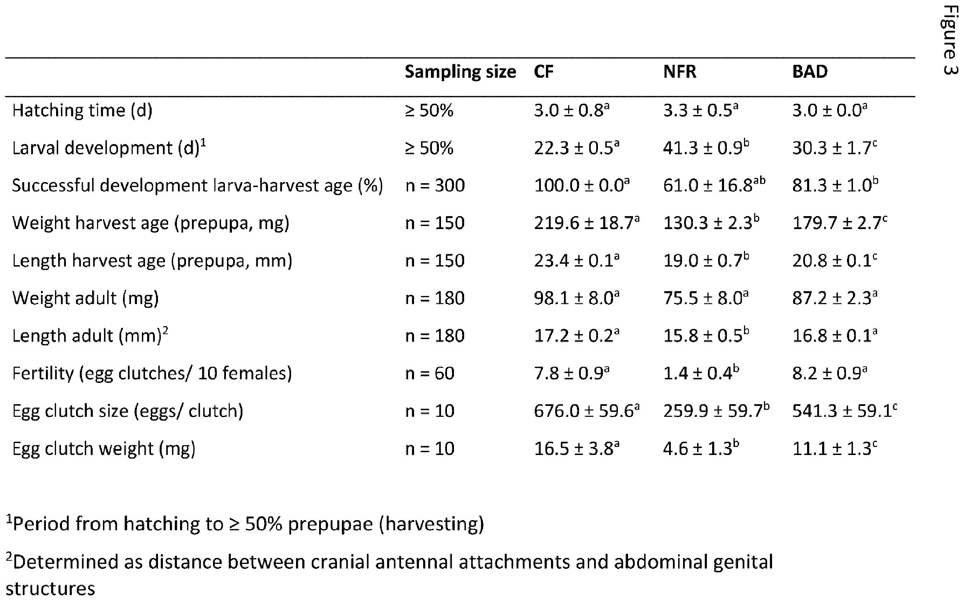

Figure 3 shows the results of an experiment in which the diet-dependent temporal and physiological development in BSF larvae were studied. In said experiment, BSF larvae were reared the three different substrates mentioned in the description of Figure 2. Data are means ±SD. The transition between two developmental stages was defined as when ≥ 50% of a population reached the next stage. Different letters (a-c) within a row indicate statistically significant differences between diets (p < 0.05; one-way ANOVA; Kaplan-Meier estimator for adult longevity).

### Example 1 - Process for the production of an insect substrate

An exemplary inventive process for the production of an insect substrate by fermentation of an empty fruit bunches (EFB) and palm kernel meal (PKM) mixture is described.

Firstly, for testing fungal growth and lignocellulose degradation in EFB, EFB was ground to a granulate with a particle size of appox. 1 mm. Said granulate was sterilized by autoclaving the mixture. Then, 50 g of the granulate were mixed with 3 mL of homogenized liquid culture of the white-rot fungus *Bjerkandera adusta.* The mixture was adjusted to a moisture content of 60 % (v/w) and fungal growth and lignocellulose degradation were confirmed visually, with degradation revealed by EFB bleaching (see Figure 1).

For producing suitable insect substrate from palm oil side products a solid-state fermentation was performed with a mixture of EFB and PKM. To this end, submerged cultures of the basidiomycete *B. adusta* were homogenized and utilized to inoculate a mixture comprising 70 wt.-% EFB and 30 wt.-% PKM at 28 °C and 60% relative humidity for 28 days (in the dark).

### Example 2 - Study of insect substrate on insect growth and development

The effects of the feed of Example 1 on insect growth and development including life history traits (exemplified on BSF) were tested and are described below.

A comparative feeding study with BSF larvae using chicken feed ("CF") as a high-quality control and an untreated mixture of EFB and PKM as a low-quality standard diet control, described as the non-fermented reference ("NFR"), was conducted. Differences between the three treatments in the development from eggs to reproducing adults were assessed to describe the quality of the newly developed insect substrate.

Hatching time: 150 mg eggs were placed in three replicate containers for each substrate and hatching time was recorded when ≥ 50% of the larvae hatched. There were no significant differences between the three substrates (see Table 1).

Larval development: Larval development, defined from hatching until harvest (i.e. time when larval biomass is highest, ≥ 50% prepupae), was significantly longer for the "BAD" substrate than for the "CF"substrate, but significantly shorter than for the "NFR" substrate (see Table 1, Figure 2). Similarly, at harvest age larval length, weight and survival rate for the "BAD" substrate was higher than for the "NFR"substrate and lower than for the "CF" substrate, although the difference for survival rate between "NFR" and "BAD" substrates was not significant (see Table 1, Figure 2).

Adults: In adult BSF there was no weight difference between the treatments. However, adults treated with the "NFR" substrate were significantly shorter than with the "BAD" and "CF" substrates, which were not different from each other (see Table 1). Reproductive parameters of the adult BSF were similarly affected. Adults reared on the "BAD" substrate produced more, larger, and heavier egg clutches than those reared on non-fermented substrate ("NFR" substrate).

Compared to chicken feed, egg clutch number wasn't different but size and weight of the clutches were smaller on the "BAD" substrate (Table 1).

Overall, BSF larvae can be successfully reared on palm oil side streams. The pretreatment of the mixture of EFB and PKM by fermentation with *B. adusta* accelerated the development and simultaneously enhanced larval weight compared to the untreated control diet.

Hence, fermentation as a pretreatment for mixtures of EFB and PKM improve its digestibility and the resulting substrate provides a suitable complete feed for insect production, exemplified by BSF rearing.

Although industrial chicken feed enables superior insect larvae growth, the provided insect substrate is more sustainable and economical. The described process for the fermentation of EFB and PKM therefore represents an effective low-tech approach for the bioconversion of high-fiber side streams by BSF larvae, allowing the production of valuable insect protein.

## Claims

1. Process for the production of an insect substrate, the process comprising or consisting of the following steps:
a) Sterilizing a substrate comprising lignocellulose;
b) Mixing the sterilized substrate comprising lignocellulose with a lignin-degrading fungus to form a solid state mixture;
c) Incubating the solid state mixture to allow a solid state fermentation, wherein enzymes produced by the fungus convert the solid state mixture to an insect substrate;
**characterized in that** the process does not comprise a step in which the substrate comprising lignocellulose is subjected to a delignification treatment involving an addition of NaOH to the substrate comprising lignocellulose.

2. Process according to the preceding claim, **characterized in that** the process does not comprise a step of
i) extracting a ligninase enzyme; and/or
ii) adding an ionic liquid; and/or
iii) adding an organic solvent.

3. Process according to one of the preceding claims, **characterized in that** the substrate comprises or consists of at least one, preferably unwashed, side stream of palm oil production, wherein the substrate preferably comprises or consists of
a) empty fruit bunches originating from a side stream of palm oil production, more preferably in an amount of 50 to 90 wt.-%, preferably 60 to 80 wt.-%, more preferably 65 to 75 wt.-%, relative to the total weight of the substrate; and/or
b) palm kernel meal originating from a side stream of palm oil production, more preferably in an amount of 10 to 50 wt.-%, preferably 20 to 40 wt.-%, more preferably 25 to 35 wt.-%, relative to the total weight of the substrate.

4. Process according to one of the preceding claims, **characterized in that** the substrate does not comprise wheat flour, preferably no carbon source being different to a side stream of palm oil production.

5. Process according to one of the preceding claims, **characterized in that** the substrate comprising lignocellulose is sterilized by steam, preferably by pressured steam at a temperature in the range of >100 °C to 121 C.

6. Process according to one of the preceding claims, **characterized in that** the substrate is
i) present in the form of particles; or
ii) processed to particles, preferably by a method selected from the group consisting of crushing, pearling, cracking, chopping, shredding, grinding, ball-milling and combinations thereof;
wherein the size of the particles is preferably in the range of 0.1 mm to 1 cm, more preferably 0.2 mm to 5 mm, even more preferably 0.5 mm to 2 mm, wherein the particle size refers to a particle size determined by microscopy.

7. Process according to one of the preceding claims, **characterized in that** the lignin-degrading fungus is selected from the group consisting of soft-rot fungi, brown-rot fungi and white-rot fungi, preferably selected from the group consisting of white-rot fungi.

8. Process according to one of the preceding claims, **characterized in that** the lignin-degrading fungus is a fungus of the division Basidiomycota, preferably a fungus of the class Agaricomycetes, more preferably
i) a fungus of the order Agaricales, even more preferably a fungus of the family Marasmiaceae, particularly a fungus of the genus *Marasmius,* especially a fungus of the species *Marasmius palmivorus*; or
ii) a fungus of the order Polyporales, even more preferably a fungus of the family Phanerochaetaceae or Cerrenaceae, particularly a fungus of the genus *Bjerkandera* or *Cerrena,* especially a fungus of the species *Bjerkandera adusta* or *Cerrena consors.*

9. Process according to one of the preceding claims, **characterized in that** the solid state mixture is adjusted to have a moisture content in the range of 40 to 80 % (v/w), preferably 50 to 70 % (v/w), more preferably 55 to 65 % (v/w), especially 60 % (v/w).

10. Process according to one of the preceding claims, **characterized in that** the mixture is incubated at a relative humidity in the range of 40 to 80 % (v/w), preferably 50 to 70 % (v/w), more preferably 55 to 65 % (v/w), especially 60 % (v/w), wherein the relative humidity is preferably adjusted by adding water, more preferably an water vapour in air, to the mixture.

11. Process according to one of the preceding claims, **characterized in that** the mixture is incubated at a temperature of 20 to 31°C, preferably 22 to 30 °C, more preferably 24 to 29°C, even more preferably 26 to 28°C, especially 28°C.

12. Process according to one of the preceding claims, **characterized in that** the mixture is incubated for at least 16 days, preferably at least 20 days, more preferably at least 24 days, even more preferably 26 to 30 days, especially 28 days.

13. Process according to one of the preceding claims, **characterized in that** incubating the solid state mixture is not implemented in
i) a flask, especially in no vessel; and/or
ii) a rotary drum bioreactor, preferably not implemented in a tray bioreactor, packed bed bioreactor, rotary drum bioreactor, swing solid state bioreactor, stirred vessel bioreactor and air solid fluidized bed bioreactor, more preferably not implemented in any bioreactor.

14. Insect substrate producible with the process according to one of the preceding claims.

15. Use of the insect substrate according to claim 14 for growing insect larvae, preferably for growing larvae of the black soldier fly.
